# EUROPEAN PATENT APPLICATION

(11) **EP 2 656 852 A2**
(43) Date of publication of application: **30.10.2013**
(21) Application number: 11849265.1
(22) Date of filing: 22.11.2011
(51) Int. Cl.: A61K 33/10, A61K 31/728, A61P 19/00

(54) **"MITSEOSTIN" PHARMACEUTICAL COMPOSITION FOR STIMULATING THE REGENERATION OF SUPPORTING TISSUE AND ARTICULAR CARTILAGE (EMBODIMENTS)**

(30) Priority: 15.12.2010 RU 2010151156
(71) Applicant: Limited Liability Company "SOZIDATEL", Moscow 127299 (RU)
(72) Inventor: BOLSHAKOVA, Anastasiya Yevgenyevna, Nizhegorodskaya obl. 607651 (RU); BORISHPOLSKIY, Andrey Leonidovich, Moscow 123154 (RU); KNYAZKIN, Gennady Jurievich, Sochi 634008 (RU); MELNIKOVA, Nina Borisovna, Nizhny Novgorod 603074 (RU); POLUKHIN, Igor Valentinovich, Moscow 111538 (RU); POLUKHIN, Oleg Valentinovich, Moscow 125080 (RU); PYANZINA, Irina Petrovna, Moscow 127411 (RU)
(74) Representative: von Füner, Nicolai
(86) International application number: PCT/RU2011/000913
(87) International publication number: WO 2012/082013

(57) **Abstract**

The invention relates to the field of medicine and pharmaceutics and, more specifically, to the creation of a pharmaceutical composition in the form of a suspension for the prevention and treatment of disorders in the physiological and reparative regeneration of tissues of the musculoskeletal system, i.e. osseous tissue and articular cartilage. The pharmaceutical compositions stimulating the regeneration of the osseous tissue and articular cartilage comprise the substance "Micellate of calcium and magnesium carbonates". The conducted trials have shown that the proposed pharmaceutical compositions have an apparent specific pharmacological activity, exhibiting a reparative and regenerative effect with respect to bone tissue in animals, and do not have a marked general toxic effect in different doses and routes of administration to laboratory animals. The compositions do not exhibit an irritant effect, and no sensitizing effect has been observed. The proposed pharmaceutical compositions are effective and safe and can be used to prevent and treat disorders in the physiological and reparative regeneration of tissues of the musculoskeletal system, *inter alia* in diabetes mellitus patients.

## Description

The invention relates to the field of medicine and pharmacy, in particular to the preparation of the pharmaceutical composition in the form of a suspension for the prevention and treatment of disorders in the physiologic and reparative regeneration of tissues of musculoskeletal system, i.e. osseous tissue and articular cartilage.

During the formation and conservation of osseous tissue a considerable part is played by calcium and phosphorus. Calcium is presented in the bones by phosphates - Ca₃(PO₄)₂ (85%), carbonates-CaCO₃ (10%), salts of organic acids - citric and lactic acids (approximately 5%). Apart from skeleton, calcium is contained in the extracellular liquid and is practically absent in the cells. The bone dense matrix along with collagen contains calcium phosphate, a crystalline mineral compound similar to hydroxyapatite Ca₁₀(PO₄)₆(OH)₂. A part of ions Ca²⁺ is substituted by ions Mg²⁺, an insignificant part of ions OH⁻ is substituted by fluorine ions improving the solidity of the bones. The mineral components of the osseous tissue are in the state of chemical balance with the calcium and phosphate ions in the blood serum. The cells of osseous tissue may accelerate the deposition or *vice versa* dissolution of mineral components in case of local changes in pH, concentration of ions Ca²⁺, HPO₄²⁻, chelating compounds (D.Mezler, 1980).

When the concentration of calcium ions decreases, the secretion of parathormone increases and osteoclasts enhance the dissolution of mineral compounds contained in the bones. The parathormone increases simultaneously the reabsorption of Ca²⁺ ions in renal tubules. Finally, the level of calcium in the blood serum increases. An increase in the amount of calcium ions causes secretion of calcitonin which reduces the concentration of Ca²⁺ ions due to the deposition of calcium in bones as a result of osteoblastic activity.

Blood plasma contains fractions of protein-bound (non-diffusing) calcium (0.9 mmol/l) and diffusing calcium: ionized (1.1-1.4 mmol/l) and non-ionized (0.35 mmol/l). [Klatter U. Disorders of Mineral and Osseous Metabolism // Therapeutic handbook of the Washington University. M.Woodley and A.Walan (editors) M., Praktika, 1995 - p. 502-601; Marshall V.J. Clinical Biochemistry / Traslation from English. - M., SP: Binome, Nevsky Dialekt, 2002 - 348 p.]

The ionized calcium is biologically active, it penetrates into the cells through membranes; the non-ionized form is bound with proteins (albumin), carbohydrates and other compounds. Depending on the pH value the binding level changes. The concentration values for the ionized calcium at the pH value of less than 7.4 vary from 4.75 to 5.20 mg/100ml, and the percentage of unbound calcium increases in case of acidosis and decreases in case of alkalosis [Tsiganenko A.Y. Clinical Biochemistry (Textbook for students of medical universities) / A.Y. Tsiganenko, V.I. Zhukov, V.V. Myasoedov, I.V. Zavgorodny. - M. Triada-X, 2002 - pp. 197-199].

The balance of the element depends on different factors (the state of the gastro-intestinal tract and urogenital system, kind and quality of food, level of vitamin D, administration of certain medicaments, etc.); it decreases with age and as the duration of menopause increases.

Disorders of calcium metabolism are accompanied by metabolic phosphates disorders and are clinically manifested in skeletal changes.

The prior art teaches a pharmaceutical composition Osteogenone (film-coated tablets of 830mg) produced by the company Pierre Fabre Medicament Production (France) in the form of ossein-hydroxyapatite complex including 75 mg non-collagen peptides/proteins, 216 mg collagen proteins (which corresponds to approximately 291 mg ossein), 178 mg calcium and 82 mg phosphorus (which corresponds to approximately 444 mg hydroxyapatite) [Mashkovsky M.D. Drugs, 15th edition. - M.: Editorial and publishing agency "Novaya Volna": Editor Umerenkov, 2008. - 692 p.].

Osteogenone has a double effect: it stimulates osteogenesis (osteoblasts due to the osteocollagenous component) and inhibits bone resorption (osteoclasts due to inorganic calcium constituent of hydroxyapatite). The preparation is used for the prevention and treatment of osteoporosis of different etiology.

The deficit of osteogenone is a low assimilability of the hydroxyapatite complex containing calcium in the hydroxyapatite in the non-ionized form. On the other hand, collagens in the pharmaceutical composition are highly susceptible to microbial contamination, so that a large amount of regulators and preservatives need to be introduced into the preparation.

The closest prior art of the proposed invention is the pharmaceutical composition of the Russian patent No. 2327478 published in 2008. Said composition is used for the prevention and treatment of disorders in the physiologic and reparative regeneration of tissue of musculoskeletal system, i.e. osseous tissue and articular cartilage. The composition comprises a number of basic ingredients of the mineral phase and extracellular matrix of supporting tissue: calcium, phosphorus, amino acids, hexosamines, and uronic acids.

The deficit of this compound is a large amount of phosphorus (20-50%) in the composition, which may first of all lead to disorders of the phosphate metabolism. Secondly, the excess concentration of phosphorus causes hyperphosphatemia which is inadmissible in case of renal insufficiency, hypoparathyroidism, pseudohypoparathyroidism, rhabdomyolysis, carcinolysis, metabolic and respiratory acidosis. Hyperphosphatemia suppresses the hydroxylation of 25-hydroxycalciferol in kidneys and, accordingly, the resorption of calcium.

Besides, calcium is contained in the preparation in the mixture of minerals in non-ionized form, which dramatically reduces its bioavailability.

The deficit of the preparation "Hondroporon" obtained according to the prior art consists in the ambiguity of the composition in terms of uronic acids. According to the prior art, uronic acids are obtained by mixing hydrolyzates of the demineralized bone matrix and hyaline cartilage of the sheep aorta in a ratio of 1:2 (multicomponent mixture of biologically active substances), which does not enable the concentration stability and purity of the uronic acids.

The purpose of the present invention is to provide a pharmaceutical composition for the prevention and treatment of disorders in physiologic and reparative regeneration of tissues of musculoskeletal system, i.e. osseous tissue and articular cartilage, which enables an improved phosphorus metabolism while normalizing the calcium and magnesium levels in the blood serum, increases the biological assimilability of calcium and reparative regeneration of bone tissue, improves the purity of components in the pharmaceutical composition.

The technical result achieved due to the realization of this invention is the earlier renewal of soft tissues in case of fractures, faster fracture healing in fractures of different types: A3 (transversal), B2 (cuneiform), C2 (segmental), complex fractures of the body and ramus of ischium, maxillofacial fractures, as well as correction of calcium, magnesium and phosphorus levels in the blood serum in case of osteoporosis and other bone diseases. Besides, the technical result consists in the increased stability of the composition as a result of the higher purity of the used components.

The claimed aim is achieved by a group of inventions by creating a pharmaceutical composition stimulating the regeneration of the osseous tissue and articular cartilage which is based on the substance "Micellate of calcium and magnesium carbonate".

| The first variant | | |
|---|---|---|
| (make-up of the composition, weight%): | | |
| | Substance "Micellate of calcium and magnesium carbonates" | 1.0-50.0 |
| | Sodium hyaluronate | 0.3-1.0 |
| | Sodium hydrophosphate dehydrate | 0.04-1.50 |
| | Potassium dihydrophosphate | 0.002-0.150 |
| | Alcohol | 1.5-2.5 |
| | Nipagin | 0.15-0.24 |
| | Fructose | 5.0-15.0 |
| | Water | the rest |

| The second variant | | |
|---|---|---|
| (make-up of the composition, weight%): | | |
| | Substance "Micellate of calcium and magnesium carbonates" | 1.0-50.0 |
| | Sodium hyaluronate | 0.3-1.0 |
| | Sodium hydrophosphate dehydrate | 0.04-1.50 |
| | Potassium dihydrophosphate | 0.002-0.150 |
| | Sodium fluoride | 0.008-0.015 |
| | Cevitamic acid | 0.10-0.25 |
| | Alcohol | 1.5-2.5 |
| | Nipagin | 0.15-0.24 |
| | Fructose | 5.0-15.0 |
| | Water | the rest |

The composition has a conventional name of Mitseostin.

Calcium and magnesium carbonates are used in the form of the substance "Micellate of calcium and magnesium carbonates" with the mass ratio of calcium and magnesium carbonates in the negatively charged suspension from 99:1 to 95:5.

The substance "Micellate of calcium and magnesium carbonates" is a complex negatively charged suspension containing calcium and magnesium carbonates in the micellar form, wherein micelles have a variable make-up:

((CaCO₃)ₙ · nCO₃²⁻ (n - x)Ca²⁺) xCa²⁺

((CaCO₃)ₙ · nHCO₃⁻ (n - x)Ca²⁺) xCa²⁺

((MgCO₃)ₙ · nCO₃²⁻ (n - x)Mg²⁺) xMg²⁺

Besides, the suspension contains manganese, copper and iron microelements (Specification 5743-001-43646913-2006).

The unique ability of calcium to be assimilated by the organism from "Micellate of calcium and magnesium carbonates" is provided by the negative charge of the micelle particle created by HCO₃and nCO₃²⁻ ions. Such particles have a high bioavailability and increase the biological assimilability of calcium.

In order to extend the preservation of the above-mentioned composition "Micellate of calcium and magnesium carbonates", whose activity is based on the negative charge of micelle particles, the pharmaceutical composition is supplemented with polysaccharide from the class of glycosaminoglycans (the salt of endogenous hyaluronic acid).

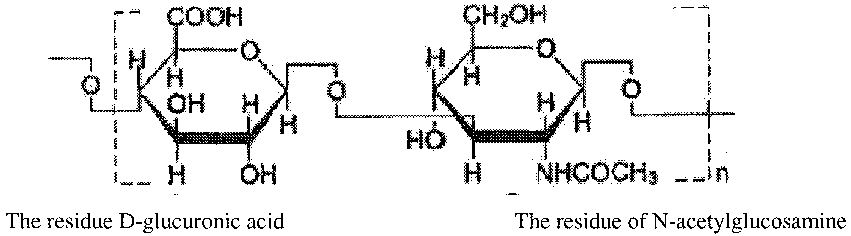

Sodium hyaluronate can be adsorbed on the micelle particle without changing its charge. In general, a micelle is electrically neutral; the charge of the particle is determined by adsorbed ions tightly bound to the micelle particle. On the picture strongly connected ions, in this case negative HCO₃⁻ and CO₃²⁻, are shown as a minus symbol inside the particle; counter-ions Ca²⁺ and Mg²⁺ are depicted as a plus symbol.

### A brief description of the figures:

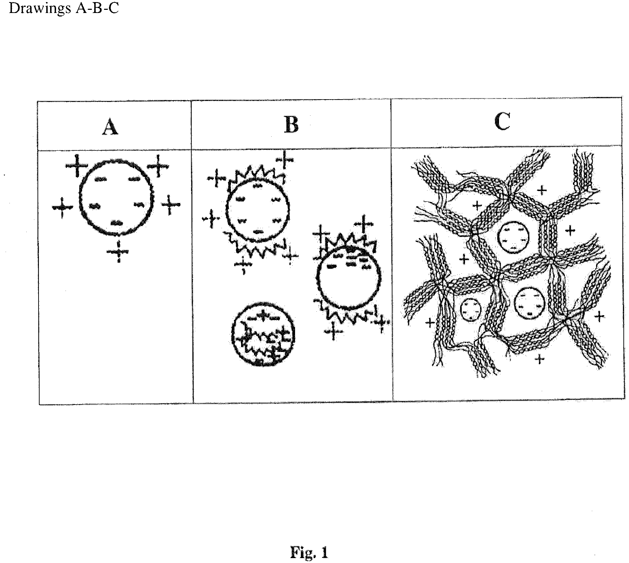

In the initial suspension "Micellate of calcium and magnesium carbonates" the total charge and zeta-potential of a micelle is zero (Figure 1A). In the absence of a polysaccharide "protection" a particle of the pharmaceutical composition may change its charge, because other components are able either to be adsorbed on the particle surface, or to react with counter-ions (Figure 1 B).

It is assumed that hyaluronic acid has a double-stranded grid structure wherein negatively charged ions and colloidal particles may be found. [Metsler D. Biochemistry. Chemical Reactions in the Live Cell / Metsler D. Translation from English, A.E. Braunstein (ed.), L.M. Ginodman, E.S. Severin. - Vol.1. - M.: Mir, 1980. - 121 p.] A unit of glucoronic acid existing in the form of a sodium salt, and N-Acetylglucosamine unit of the hyaluronic acid are usually electrically neutral and enable the capture of negatively charged particles of the suspension "Micellate of calcium and magnesium carbonates" thereby preventing the interaction with other components of the composition (Figure 1 C).

On the other hand, hyaluronic acid structures the suspension and reveals an enveloping and regenerating action, thereby protecting the gastrointestinal system [Goa K.L., Benfield P. Hyaluronic Acid. A Review of its Pharmacology and Use as a Surgical Aid in Ophthalmology and its Therapeutic Potential in Joint Disease and Wound Healing // Drugs. - 1994. - V.47. - No.3. -P. 536-566].

Fluoride ions are introduced into the pharmaceutical composition in order to reduce the resorption of osseous tissue due to the stimulation of osteoblasts, if necessary. In order to prevent the adverse influence of fluorine, one of the proposed embodiments comprises apart from fluoride ion cevitamic acid.

Fructose as an auxiliary substance has the function of a sweetener, due to which the pharmaceutical composition may be recommended to patients having diabetes mellitus.

A nipagin alcoholic solution was selected as a preservative. It is well-known that nipagin which is a methyl ether of parahydroxybenzoic acid (paraben), is recommended due to its low toxicity for preserving medical preparations for inner administration, in particular hormones. In addition, paraben can be effectively combined with glycosaminoglycans (hyaluronic acid).

Embodiments of the composition:

### Example 1.

From 0.3 to 1.0 g sodium hyaluronate is introduced into a reactor with the stirring mechanism containing 30 ml of water. While stirring 1 to 50 g of substance "Micellate of calcium and magnesium carbonates" are continuously added, followed by 5 - 15 g fructose. After that 0.04-1.5 g sodium hydrophosphate dehydrate, 0.002-0.15 g potassium dihydrogen phosphate, 1.5-2.5 g alcohol and 0.15-0.24 g nipagin are added. Finally, water is added to the obtained mixture until it reaches 100 g.

The make-up of the composition is as follows, weight%:

| | |
|---|---|
| Substance "Micellate of calcium and magnesium carbonates" | 1.0-50.0 |
| Sodium hyaluronate | 0.3-1.0 |
| Sodium hydrophosphate dehydrate | 0.04-1.50 |
| Potassium dihydrophosphate | 0.002-0.150 |
| Sodium fluoride | 0.008-0.015 |
| Cevitamic acid | 0.10-0.25 |
| Alcohol | 1.5-2.5 |
| Nipagin | 0.15-0.24 |
| Fructose | 5.0-15.0 |
| Water | The rest up to 100 |

The compositions are in the form of a suspension.
The make-up of the proposed compositions is given in table 1.

**Table 1**

| The make-up of the proposed compositions | | | | | | | | | |
|---|---|---|---|---|---|---|---|---|---|
| Example number | 1 | 2 | 3 | 4 | 5 | 6 | 7 | 8 | Closest prior art*, % |
| The amount of the water suspension % | | | | | | | | | |
| Substance "Micellate of calcium and magnesium carbonates" | 1 | 1 | 10 | 20 | 35 | 45 | 50 | 50 | Calcium compound 30-70 |
| Sodium hyaluronate | 1.0 | 0.3 | 1.0 | 0.6 | 0.7 | 0.7 | 0.3 | 0.6 | Uronic acid 2-10 |
| Sodium hydrophosphate dihydrate | 0.04 | 0.05 | 0.08 | 1.00 | 1.20 | 1.30 | 1.40 | 1.50 | phosphorus compound 20-50 |
| Potassium dihydrophosphate | 0.002 | 0.020 | 0.040 | 0.050 | 0.050 | 0.100 | 0.130 | 0.150 | - |
| Sodium fluoride | 0.008 | 0.009 | 0.010 | 0.000 | 0.011 | 0.013 | 0.014 | 0.015 | - |
| Cevitamic acid | 0.10 | 0.15 | 0.18 | 0.00 | 0.20 | 0.23 | 0.24 | 0.25 | - |
| Alcohol | 1.5 | 1.7 | 1.8 | 2.0 | 2.1 | 2.3 | 2.4 | 2.5 | - |
| Nipagin | 0.15 | 0.17 | 0.18 | 0.20 | 0.21 | 0.22 | 0.23 | 0,24 | - |
| Fructose | 5 | 7 | 9 | 10 | 11 | 13 | 14 | 15 | |

| | | | | | | | | | |
|---|---|---|---|---|---|---|---|---|---|
| * Patent of the Russian Federation 2327478 | | | | | | | | | |

Example No. 4 is the preferable one.

The pre-clinical study on the efficacy of the pharmacological activity and safety of the suggested composition was performed in the Central Scientific Research Laboratory of the State Educational Institution for Higher Professional Training "Nizhny Novgorod State Medical Academy of the Ministry of Public Health of the Russian Federation" pursuant to the requirements of the Order of the Russian Health Service of June 19, 2003 No. 267 "About the Approval of the Regulations of the Laboratory Practice in the Russian Federation", as well as according to the Guidebook of the Experimental (Pre-clinical) Study of the New Pharmaceutical Substances. - M.: Medecine, 2005. - 832 p. The treatment and care of animals were performed in accordance with the international regulations of care and use of laboratory animals. - Guide for Care and Use of Laboratory Animals. ILAR publication, 1996, National Academy Press.

The specific pharmacological activity has been studied while simulating the glucocorticoid osteoporosis in rats received from the farm "Stolbovaya" State Institution Scientific Centre for Biological Medical Equipment of the Russian Academy of Medical Sciences by means of daily intraperitoneal administration of hydrocortisone acetate to the animals.

The conducted trials have proved that the proposed pharmaceutical compositions exhibit a marked specific pharmacological activity, as they show a reparative and regenerative effect to the osseous tissue of the animals and do not produce an essential systemic toxic impact at different doses and routes of administration to experimental animals. The compositions do not exhibit an irritant effect, and no sensitizing effect has been observed.

The proposed pharmaceutical compositions are potent and safe medicaments and may be used for the prevention and treatment of disorders in physiologic and reparative regeneration of tissues of musculoskeletal system, *inter alia* in diabetes mellitus patients.

## Claims

1. A pharmaceutical composition stimulating the regeneration of the osseous tissue and articular cartilage comprising the compounds of calcium, phosphorus, a biologically active substance on the basis of uronic acids and auxiliaries, **characterized in that** the calcium compound is represented by the substance "Micellate of calcium and magnesium carbonates", the phosphorus compound is represented by sodium hydrophosphate dihydrate and potassium dihydrophosphate, the biologically active substance is represented by sodium hyaluronate, nipagin, alcohol, fructose and water in the following ratio (weight%):
| | |
|---|---|
| Substance "Micellate of calcium and magnesium carbonates" | 1.0-50.0 |
| Sodium hyaluronate | 0.3-1.0 |
| Sodium hydrophosphate dehydrate | 0.04-1.50 |
| Potassium dihydrophosphate | 0.002-0.150 |
| Alcohol | 1.5-2.5 |
| Nipagin | 0.15-0.24 |
| Fructose | 5.0-15.0 |
| Water | the rest |

2. A pharmaceutical composition stimulating the regeneration of the osseous tissue and articular cartilage comprising the compounds of calcium, phosphorus, a biologically active substance on the basis of uronic acids and auxiliaries, **characterized in that** the calcium compound is represented by the substance "Micellate of calcium and magnesium carbonates", the phosphorus compound is represented by sodium hydrophosphate dihydrate and potassium dihydrophosphate, the biologically active substance is represented by sodium hyaluronate, sodium fluoride, cevitamic acid, alcohol, nipagin, fructose and water in the following ratio (weight%):
| | |
|---|---|
| Substance "Micellate of calcium and magnesium carbonates" | 1.0-50.0 |
| Sodium hyaluronate | 0.3-1.0 |
| Sodium hydrophosphate dehydrate | 0.04-1.50 |
| Potassium dihydrophosphate | 0.002-0.150 |
| Sodium fluoride | 0.008-0.015 |
| Cevitamic acid | 0.10-0.25 |
| Alcohol | 1.5-2.5 |
| Nipagin | 0.15-0.24 |
| Fructose | 5.0-15.0 |
| Water | the rest |
